# EUROPEAN PATENT SPECIFICATION

(11) **EP 3 675 844 B1**
(45) Date of publication and mention of the grant of the patent: **08.03.2023**
(21) Application number: 18758625.0
(22) Date of filing: 24.08.2018
(51) Int. Cl.: A61K 31/14, A61K 31/155, A61K 31/352, A61K 31/404, A61K 31/445, A61K 31/454, A61K 31/495, A61K 31/519, A61K 31/5415, A61K 31/553, A61P 19/10

(54) **BMP-MIMETICS**
BMP-MIMETIK
MIMÉTIQUES DE BMP

(30) Priority: 01.09.2017 EP 17189054
(43) Date of publication of application: 08.07.2020
(60) Divisional application: 23152770.6
(73) Proprietor: Christian-Albrechts-Universität zu Kiel, 24118 Kiel (DE)
(72) Inventor: SCHADE, Dennis, 24114 Kiel (DE); HALVER, Jonas, 44227 Dortmund (DE)
(74) Representative: Taruttis, Stefan Georg
(86) International application number: PCT/EP2018/072884
(87) International publication number: WO 2019/042889

(56) References cited:
- WO-A2-2005/079250
- WO-A2-2007/095161
- Vrijens: "Identification of Small Molecule Activators of BMP Signaling", PLoS ONE 8(3): e59045, 19 March 2013 (2013-03-19), XP055452356, Retrieved from the Internet: URL:http://journals.plos.org/plosone/artic le/file?id=10.1371/journal.pone.0059045&ty pe=printable [retrieved on 2018-02-19]
- JAMIE R. GENTHE ET AL: "Ventromorphins: A New Class of Small Molecule Activators of the Canonical BMP Signaling Pathway", ACS CHEMICAL BIOLOGY, vol. 12, no. 9, 29 August 2017 (2017-08-29), pages 2436-2447, XP055452348, US ISSN: 1554-8929, DOI: 10.1021/acschembio.7b00527
- TAKAYUKI YONEZAWA ET AL: "Harmine promotes osteoblast differentiation through bone morphogenetic protein signaling", BIOCHEMICAL AND BIOPHYSICAL RESEARCH COMMUNICATIONS, vol. 409, no. 2, 2011, pages 260-265, XP028375635, ISSN: 0006-291X, DOI: 10.1016/J.BBRC.2011.05.001 [retrieved on 2011-05-06]
- WU XU ET AL: "A small molecule with osteogenesis-inducing activity in multipotent mesenchymal progenitor cells", JOURNAL OF THE AMERICAN CHEMICAL SOCIETY, AMERICAN CHEMICAL SOCIETY, US, vol. 124, no. 49, 1 January 2002 (2002-01-01), pages 14520-14521, XP003005264, ISSN: 0002-7863, DOI: 10.1021/JA0283908
- OHBA ET AL: "A novel osteogenic helioxanthin-derivative acts in a BMP-dependent manner", BIOCHEMICAL AND BIOPHYSICAL RESEARCH COMMUNICATIONS, ELSEVIER, AMSTERDAM, NL, vol. 357, no. 4, 10 May 2007 (2007-05-10), pages 854-860, XP022106933, ISSN: 0006-291X, DOI: 10.1016/J.BBRC.2007.03.173
- STEFANO MORO ET AL: "Combined Target-Based and Ligand-Based Drug Design Approach as a Tool To Define a Novel 3D-Pharmacophore Model of Human A 3 Adenosine Receptor Antagonists: Pyrazolo[4,3- e ]1,2,4-triazolo[1,5- c ]pyrimidine Derivatives as a Key Study", JOURNAL OF MEDICINAL CHEMISTRY, vol. 48, no. 1, 1 January 2005 (2005-01-01), pages 152-162, XP055453211, ISSN: 0022-2623, DOI: 10.1021/jm049662f
- BILODEAU M L ET AL: "Adenosine signaling promotes neuronal, catecholaminergic differentiation of primary neural crest cells and CNS-derived CAD cells", MOLECULAR AND CELLULAR NEUROSCIEN, SAN DIEGO, US, vol. 29, no. 3, 1 July 2005 (2005-07-01), pages 394-404, XP004930780, ISSN: 1044-7431, DOI: 10.1016/J.MCN.2005.03.006
- A.M. TESCH ET AL: "Endogenously produced adenosine regulates articular cartilage matrix homeostasis: enzymatic depletion of adenosine stimulates matrix degradation", OSTEOARTHRITIS AND CARTILAGE., vol. 12, no. 5, 1 May 2004 (2004-05-01), pages 349-359, XP055453306, GB ISSN: 1063-4584, DOI: 10.1016/j.joca.2004.01.002
- ARÁNZAZU MEDIERO ET AL: "Direct or indirect stimulation of adenosine A 2A receptors enhances bone regeneration as well as bone morphogenetic protein-2", THE FASEB JOURNAL, vol. 29, no. 4, 1 April 2015 (2015-04-01), pages 1577-1590, XP055453310, US ISSN: 0892-6638, DOI: 10.1096/fj.14-265066

## Description

### FIELD OF THE INVENTION

The present invention lies in the field of medicinal chemistry and chemical biology and relates to a method for differentiating, transdifferentiating or reprogramming a cell by contacting a cell with a compound as defined in the present invention. The invention further relates to said compounds as defined in the present invention for use in the treatment of osteoporosis, bone fracture, osteonecrosis, osteoarthritis, rheumatoid arthritis, spondylosis, anterior lumbar fusion or long bone fracture and to a method for determining bone morphogenetic protein (BMP) mimetic activity of a compound of interest.

### BACKGROUND OF THE INVENTION

Bone morphogenetic proteins (BMPs) are cytokines with diverse functions belonging to the ligands of the TGFβ superfamily. They were originally named after their ability to promote the formation of bones *in vivo* (Chen et al., 2004, Reddi et al., 2009). Based on their sequence homology, BMPs are divided into four groups: BMP2/4, BMP5/6/7 /8a /8b, BMP9/10, and BMP12/13/14 (Bragdon et al., 201 1, Begam et al., 2017). Their influence on the differentiation of stem cells is not limited to the stimulation of osteogenesis, but is also found in the development and homeostasis of a large number of human organ systems (Wagner et al., 2010, Lowery et al., 2016) and their activity is associated with several pathologies (Salazar et al., 2016, Morrell et al., 2016, Lowery et al., 2010).

After BMPs are bound to the transmembrane BMP receptor type I, this receptor recruits the type II receptor that is thereby activated. The active type I receptor possesses an intracellular serine/threonine kinase that phosphorylates the intracellular effectors Smad1 and Smad5, which leads to their separation from the receptor complex. The phosphorylated Smad1 and Smad5 then form a complex with Smad4 and translocate into the nucleus to activate the transcription of their target genes (Derynck et al., 1998, Massague et al., 2000). The BMP/Smad pathway is also negatively regulated by structurally and functionally different Smad effector proteins of the subfamily "inhibitory Smads" (= Smad 6 and Smad 7). The Smad effector proteins are subsequently degraded, whereby the ubiquitin-ligase Smurf1 plays a decisive role.

While there is a plurality of inhibitors of the BMP signal transduction cascade, only a few activators are known. Primarily the activation of the signal pathway in the absence of BMP ligands is a great challenge.

BMP mimetic compounds may have a huge potential, particularly as drugs with therapeutic purposes and as molecules that can be used in biotechnological applications.

Osteopenic diseases such as osteoporosis, bone fractures, inflammation-related bone destruction, osteoarthritis or rheumatoid arthritis are an enormous challenge for the health and economic system as well as society. Compounds having BMP mimetic activity are a promising tool for the treatment of the above-mentioned diseases.

As concerns the use of BMP mimetic compounds in biotechnology, the BMP signaling pathway is a major regulator of developmental biology, various BMPs are important for the use of stem cell-based technologies. They can be used specifically to influence the fate of stem and precursor cells. On the one hand, this is the so-called "directed differentiation" of embryonic and/or inductive pluripotent stem cells into desired specialized target cells and tissues. In this context, BMPs are used in the induction of mesoderm and are thus used for the generation of all cell/tissue types which are derived from the mesoderm: a) cardiovascular precursor cells and myoblasts, which later form the cells of the blood vessels, the heart muscle and the skeletal muscle; b) hematopoietic stem cells, which form all cells of the blood; c) mesenchymal stem cells (MSCs), which are the starting point for cells of adipose tissue, bone and cartilage.

The use of BMP4, for example, is particularly well-suited for the targeted generation of cardiac precursor cells and myocardial cells from human stem cells (Burridge et al., 2014, Zhang et al., 2015, Rao et al., 2016).

In addition to the directed differentiation from pluripotent stem cells, BMPs (especially BMP4) can be used in the so-called "transdifferentiation" of fibroblasts to form cardiac muscle cells. Such methods have been described in the art (Wang et al 2014, Cao et al 2016, Zhang et al., 2016). The transdifferentiation is a process in which not necessarily requires a pluripotent stem cell, but rather terminally differentiated cells (such as fibroblasts) are intermediately transformed into a multipotent state (de-differentiation), in order to subsequently differentiate into the desired target cell.

Hence, there is need in the art for improved methods for differentiating, transdifferentiating or reprogramming cells, methods for determining bone morphogenetic protein (BMP) mimetic activity of a compound of interest and the treatment of osteoporosis, bone fracture, osteonecrosis, osteoarthritis, rheumatoid arthritis, spondylosis, anterior lumbar fusion or long bone fracture.

### SUMMARY OF THE INVENTION

It is an object of the present invention to meet the above need by providing methods for differentiating, transdifferentiating or reprogramming a cell by contacting said cell with a compound as defined in the present invention. Further the present invention provides compounds as defined herein for use in the treatment of osteoporosis, bone fracture, osteonecrosis, osteoarthritis, rheumatoid arthritis, spondylosis, anterior lumbar fusion or long bone fracture and a method for determining bone morphogenetic protein (BMP) mimetic activity of a compound of interest. Surprisingly, the present inventors have found a narrow time frame during differentiation of murine embryonic stem cells to cardiac muscle cells in which BMP activity has a cardiomyogenesis blocking effect. When BMP activity is blocked during said specific time frame (for example by selective BMP inhibitors, such as DMH1), the tested cells show expression of cardiac muscle cell specific genes after at least eight days of treatment. This expression (or the lack of expression) can be used as readout for BMP activity. Compounds that should be tested for BMP mimetic activity are applied to the cells in parallel with the BMP inhibitor. When no expression of cardiac muscle cell specific genes is observed after all steps of the differentiation protocol have been applied to the cells, the compound is believed to exhibit cardiomyogenesis blocking and BMP mimetic activity.

By using the above-described screening assay, the present inventors identified ten compounds that have BMP mimetic activity. Such compounds can be used for biotechnological applications, such as differentiation, transdifferentiation or reprogramming applications, or for the treatment of diseases, such as osteoporosis, bone fracture, osteonecrosis, osteoarthritis, rheumatoid arthritis, spondylosis, anterior lumbar fusion or long bone fracture.

In a first aspect, the present invention relates to an *in vitro* method for differentiating, transdifferentiating or reprogramming a cell, said method comprising: contacting said cell with the compound 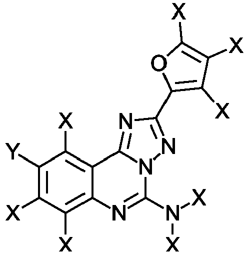 wherein
X is H;
Y is halogen.

In an embodiment of the invention, the compound is

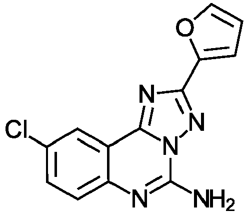

In various embodiments,
(I) said method is a method to differentiate a cell and
   (a) the cell is a pluripotent stem cell; and/or
   (b) the cell differentiates into a multipotent cell, preferably a cardiac progenitor cell, myoblast, hematopoietic stem cell or mesenchymal stem cell; and/or
   (c) the differentiation further comprises contacting the cell with at least one compound supporting differentiation;
(II) said method is a method to transdifferentiate a cell and
   (a) the cell is a fibroblast; and/or
   (b) the cell transdifferentiates into a cardiac muscle cell; and/or
   (c) the transdifferentiation further comprises contacting the cell with at least one compound supporting transdifferentiation; or
(III) said method is a method to reprogram a cell and
   (a) the cell is a fibroblast; and/or
   (b) the cell reprograms into a pluripotent stem cell; and/or
   (c) the reprogramming further comprises contacting the cell with at least one compound selected from the group of Oct-4, Sox-2, Klf-4 or c-Myc.

In a second aspect, the present invention is directed to a compound or a pharmaceutically acceptable salt, solvate or prodrug thereof as described herein for use in the treatment of osteoporosis, bone fracture, osteonecrosis, osteoarthritis, rheumatoid arthritis, spondylosis, anterior lumbar fusion or long bone fracture, wherein said prodrug is a phosphoric acid ester linked via a one-carbon-bridge.

### BRIEF DESCRIPTION OF THE DRAWINGS

The invention will be better understood with reference to the detailed description when considered in conjunction with the non-limiting examples and the accompanying drawings.

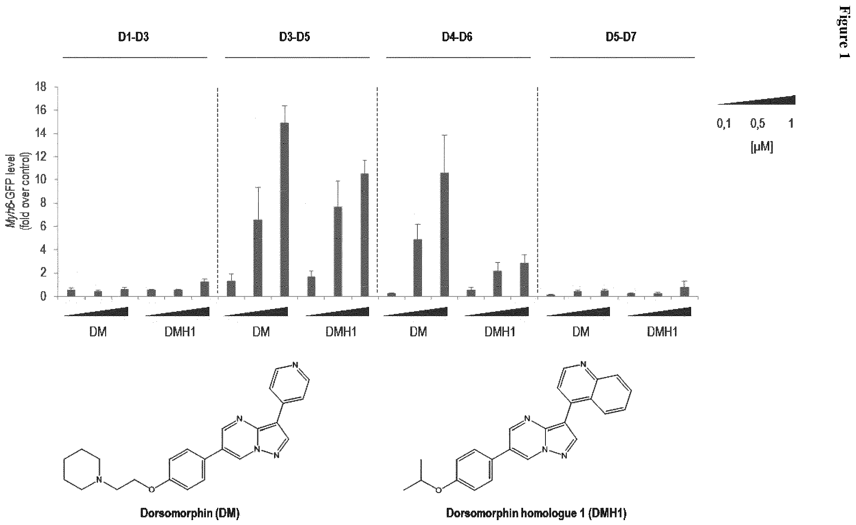

**Figure 1** shows data to establish an assay system in murine embryonic stem cells (mESCs) to identify bone morphogenetic protein (BMP) mimetic compounds. The BMP inhibitors dorsomorphine (DM) and DMH1 demonstrate depending on their individual selective inhibitory profile different pro-cardiac activity.

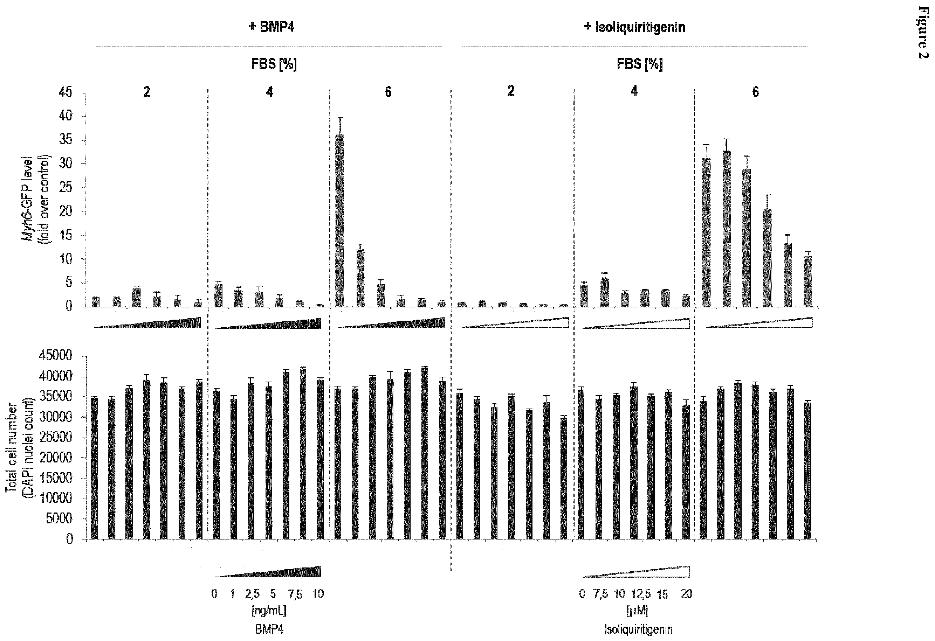

**Figure 2** shows that the DMH1 induced cardiomyogenesis, which is induced between days 3 and 4, can be completely antagonized by BMP4, whereas isoliquirtigenin only demonstrates an incomplete inhibition of the described effect.

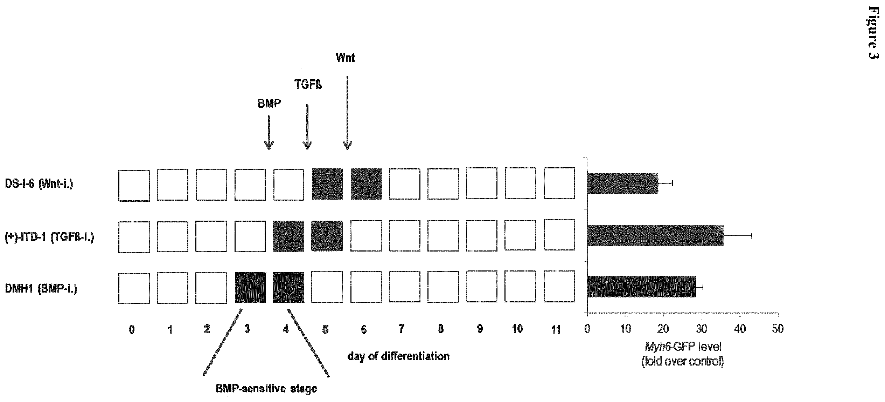

**Figure 3** shows a summary of the pro-cardiac effects of BMP inhibition (DMH1), TGFβ inhibition (DHP-1) and Wnt/β-catenin inhibition (IWR type inhibitor DS-I-6).

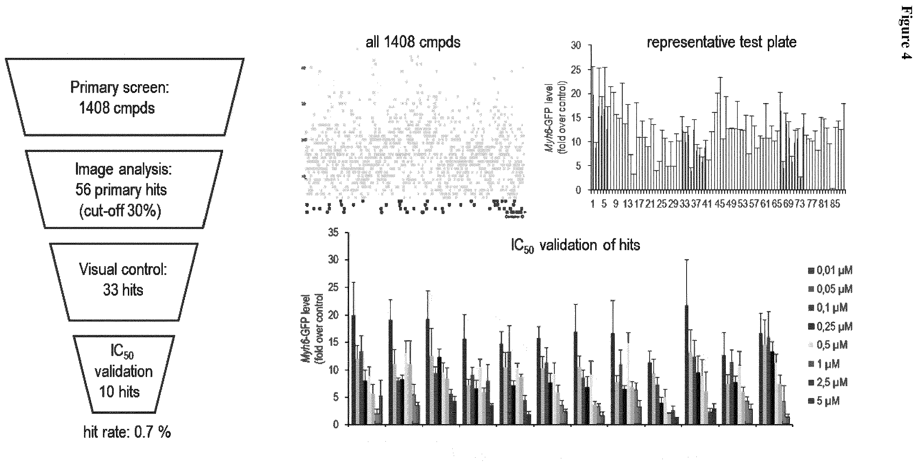

**Figure 4** shows that a high content-screening of the LOPAC compound library using the screening assay of the invention identifies BMP mimetic compounds. A workflow is provided including an overview of the whole screen (1408 compounds) and a representative screening result of a 384 well plate testing 90 compounds. For 33 hits the IC₅₀ value was determined, wherein 10 compounds demonstrates a dosage dependent BMP mimetic activity.

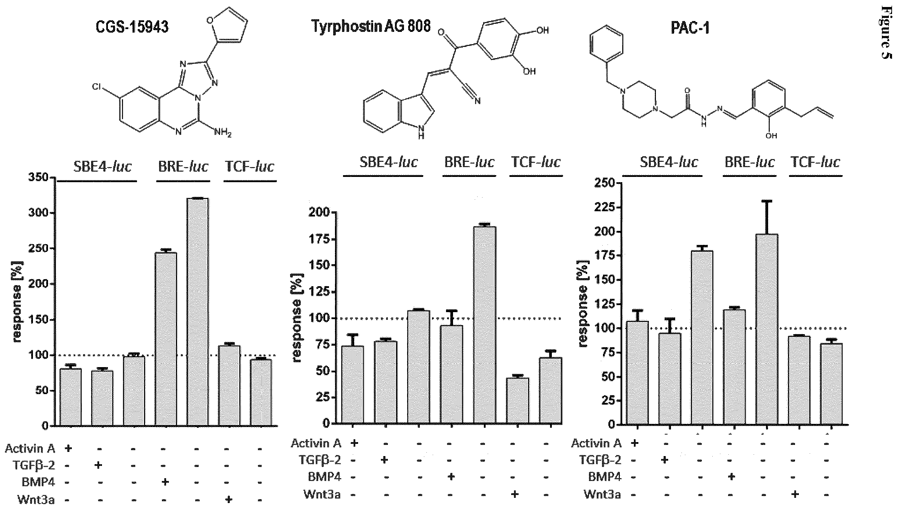

**Figure 5** shows the signal pathway selectivity of three of the ten compounds identified as hits in the screening assay of the invention. HEK 293T cells are transiently transfected with the following reporter constructs: SBE4-luc (Smad4-binding element luciferase reporter construct), BRE-luc (BMP-responsive element luciferase reporter construct), TCF-luc (TCF/LEF transcriptional response element). The signal pathways were stimulated with 10 ng/mL of the corresponding growth factor and the hit compounds were tested at a concentration of 5 µM.

### DETAILED DESCRIPTION OF THE INVENTION

The present inventors surprisingly found a time frame during differentiation of stem cells in which BMP activity alone can block the further differentiation of the stem cell (cardiomyogenesis blocking activity). This time frame can be used to screen for compounds with BMP mimetic activity. Based on their observation, the present inventors have established a high content screening assay to screen for BMP mimetics. By using their screening assay and a compound library of 1408 compounds, the present inventors found ten compounds that exhibit BMP mimetic activity.

Thus, in a first aspect, the present invention relates to a method for differentiating, transdifferentiating or reprogramming a cell, said method comprising:
contacting said cell with the compound

The terms "differentiation" or "direct differentiation", as interchangeably used herein, refers to a process of converting pluripotent or multipotent cell to a desired terminally differentiated cell (for example a somatic cell). For example, differentiation refers to a process by which precursor or progenitor cells differentiate into specific cell types such as osteocytes, cardiac cells and chondrocytes. The term "inducing differentiation", as used herein, is taken to mean causing a stem cell to develop into a specific differentiated cell type as a result of a direct or intentional influence on the stem cell. Influencing factors can include cellular parameters such as ion influx, a pH change and/or extracellular factors, such as secreted proteins or small molecule compounds (such as the compounds described herein), growth factors and cytokines that regulate and trigger differentiation. It may include culturing the cell to confluence and may be influenced by cell density.

The term "transdifferentiation", as used herein, refers to the process that starts with de-differentiation of terminally differentiated cell (somatic cell A, unipotent) to a multipotent state, followed by differentiation to a different terminally differentiated cell type (somatic cell B). Said process does not comprise a pluripotent state. Thus, transdifferentiation describes the conversion of a non-stem cell into a different type of cell. Transdifferentiation is a type of metaplasia, which is the replacement of one differentiated cell type with another differentiated cell type. It is generally caused by some sort of abnormal stimulus such as a dramatic change in the cell's environment. Cell types are distinct morphological or functional forms of cells.

The terms "reprogramming" and "reprogram", as used herein, refer to the dedifferentiation of terminally differentiated cells (such as somatic cells) to a) pluripotent cells or b) multipotent cells (= part of transdifferentiation). Thus, reprogramming is a process involving the re-differentiation or dedifferentiation of a cell having the morphological and functional characteristics representative of a certain class of cells, into a cell having known morphological and functional characteristics of a different class of cells. Thus, for example, fibroblasts may be reprogrammed to pluripotent stem cells.

The term "dedifferentiate" or "dedifferentiation", as used herein, refers to the process by which lineage committed cells (e.g. myoblasts or osteoblasts) reverse their lineage commitment and become precursor or progenitor cells (i.e. multipotent or pluripotent stem cells).

"Redifferentiation", as used herein, refers to a process by which a group of once differentiated cells return to their original specialized form.

The above terms are defined in more detail in Xie, M. et al. (Xie, M. et al. (2017) Acc Chem Res.;50(5):1202-1211).

The term "pharmaceutically acceptable salt", as used herein, is meant to include salts of active compounds, which are prepared with relatively nontoxic acids or bases, depending on the particular substituent moieties found on the compounds described herein. When compounds of the disclosure contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, anmonium, organic amino, or magnesium salt, or a similar salt. When compounds of the disclosure contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like formic, acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., "Pharmaceutical Salts", Journal of Pharmaceutical Science, 1977, 66, 1-19). Certain specific compounds of the disclosure contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms in certain physical properties, such as solubility in polar solvents.

Thus, according to certain embodiments, a pharmaceutically salt of a compound as disclosed herein, may be the salt of 1-hydroxy-2-naphthoic acid, 2,2-dichloroacetic acid, 2-hydroxyethanesulfonic acid, 2-oxoglutaric acid, 4-acetamidobenzoic acid, 4-aminosalicylic acid, acetic acid, adipic acid, ascorbic acid (L), aspartic acid (L), benzenesulfonic acid, benzoic acid, camphoric acid (+), camphor-10-sulfonic acid (+), capric acid (decanoic acid), caproic acid (hexanoic acid), caprylic acid (octanoic acid), carbonic acid, cinnamic acid, citric acid, cyclamic acid, dodecylsulfuric acid, ethane-1,2-disulfonic acid, ethanesulfonic acid, formic acid, fumaric acid, galactaric acid, gentisic acid, glucoheptonic acid (D), gluconic acid (D), glucuronic acid (D), glutamic acid, glutaric acid, glycerophosphoric acid, glycolic acid, hippuric acid, hydrobromic acid, hydrochloric acid, isobutyric acid, lactic acid (DL), lactobionic acid, lauric acid, maleic acid, malic acid (L), malonic acid, mandelic acid (DL), methanesulfonic acid, naphthalene-1,5-disulfonic acid, naphthalene-2-sulfonic acid, nicotinic acid, nitric acid, oleic acid, oxalic acid, palmitic acid, pamoic acid, phosphoric acid, propionic acid, pyroglutamic acid (-L), salicylic acid, sebacic acid, stearic acid, succinic acid, sulfuric acid, tartaric acid (+ L), thiocyanic acid, toluene sulfonic acid (p), or undecylenic acid

Certain compounds of the disclosure can exist in unsolvated forms as well as solvated forms ("solvates"), including hydrated forms. In general, the solvated forms are equivalent to unsolvated forms and are encompassed within the scope of the disclosure. Certain compounds of the disclosure may exist in multiple crystalline or amorphous forms. In general, all physical forms are equivalent for the uses contemplated by and are intended to be within the scope of the disclosure.

Certain compounds of the disclosure possess asymmetric carbon atoms (optical or chiral centers) or double bonds; the enantiomers, racemates, diastereomers, tautomers, geometric isomers, stereoisometric forms that may be defined, in terms of absolute stereochemistry, as (R)- or (S)-or, as (D)- or (L)- for amino acids, and individual isomers are encompassed within the scope of the disclosure. The compounds of the disclosure do not include those, which are known in art to be too unstable to synthesize and/or isolate. The disclosure is meant to include compounds in racemic and optically pure forms. Optically active (R)- and (S)-, or (D)-and (L)-isomers may be prepared using chiral synthons or chiral reagents, or resolved using conventional techniques. When the compounds described herein contain olefinic bonds or other centers of geometric asymmetry, and unless specified otherwise, it is intended that the compounds include both E and Z geometric isomers.

The term "tautomer," as used herein, refers to one of two or more structural isomers which exist in equilibrium and which are readily converted from one isomeric form to another. It will be apparent to one skilled in the art that certain compounds of the disclosure may exist in tautomeric forms, all such tautomeric forms of the compounds being within the scope of the disclosure.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the disclosure.

Unless otherwise stated, structures depicted herein are also meant to include compounds, which differ only in the presence of one or more isotopically enriched atoms. For example, compounds having the present structures except for the replacement of a hydrogen by a deuterium or tritium, or the replacement of a carbon by 13C- or 14C -enriched carbon are within the scope of the disclosure.

The term "prodrug", as used herein, refers to a compound, which is in a prodrug form. Prodrugs of the compounds described herein are those compounds that readily undergo chemical changes under physiological conditions to provide the compounds of the disclosure.

Prodrug forms of the herein disclosed compounds are designed to improve their physicochemical properties (e.g. solubility, hydrophilicity, stability) and pharmacokinetic behavior (e.g. absorption, distribution, metabolism, excretion and toxicity). Prodrugs of the herein disclosed compounds can be designed for enrichment in the target cells, tissues or organs (e.g. bone, cartilage, bone marrow, heart etc.).

Additionally, prodrugs can be converted to the compounds of the disclosure by chemical or biochemical methods in an *ex vivo* environment. For example, prodrugs can be slowly converted to the compounds of the disclosure when placed in a transdermal patch reservoir with a suitable enzyme or chemical reagent.

For example, a prodrug form of the herein disclosed compounds can be a phosphoric acid ester linked to the parent molecule via a one-carbon-bridge (e.g., methylene group or alkylmethyl group linker). The phosphate ester can undergo spontaneous chemical degradation or be hydrolytically cleaved by hydrolases (e.g., esterases, phosphatases), rendering a semiacetal or semiketal (e.g., O,O-, O,N-, O,S-semiacetal or semiketal) that further degrades chemically to release the bioactive parent compound.

The term "cell", as used herein, can refer to a single and/or isolated cell or to a cell that is part of a multicellular entity such as a tissue, an organism or a cell culture. In other words the methods can be performed *in vivo, ex vivo* or *in vitro.* The cell is a eukaryotic cell. A eukaryotic cell, as used herein, refers to any cell of a multi-cellular eukaryotic organism, including cells from animals like vertebrates. Preferably, the cell is a mammalian cell. The term "mammalian cell", as used herein, is well known in the art and refers to any cell belonging to or derived from an animal that is grouped into the class of mammalia. Cells of different mammalian subclasses such as prototheria or theria may be used. For example, within the subclass of theria, preferably cells of animals of the infraclass eutheria, more preferably of the order primates, artiodactyla, perissodactyla, rodentia and lagomorpha are used in the methods of the invention. Furthermore, within a species one may choose a cell to be used in the methods of the invention based on the tissue type and/or capacity to differentiate equally depending on the goal to be achieved by modifying the genome via transducing a target cell according to the method of the invention. There are three basic categories of cells that make up the mammalian body: germ cells, somatic cells and stem cells. A germ cell is a cell that gives rise to gametes and thus is continuous through the generations. Stem cells can divide and differentiate into diverse specialized cell types as well as self-renew to produce more stem cells. In mammals there are two main types of stem cells: embryonic stem cells and adult stem cells. Somatic cells include all cells that are not a gametes, gametocytes or undifferentiated stem cells. The cells of a mammal can also be grouped by their ability to differentiate. A totipotent (also known as omnipotent) cell is a cell that is able to differentiate into all cell types of an adult organism including placental tissue such as a zygote (fertilized oocyte) and subsequent blastomeres, whereas pluripotent cells, such as embryonic stem cells, cannot contribute to extraembryonic tissue such as the placenta, but have the potential to differentiate into any of the three germ layers endoderm, mesoderm and ectoderm. Multipotent progenitor cells have the potential to give rise to cells from multiple, but limited number of cell lineages. Further, there are oligopotent cells that can develop into only a few cell types and unipotent cells (also sometimes termed a precursor cell) that can develop into only one cell type. There are four basic types of tissues: muscle tissue, nervous tissue, connective tissue and epithelial tissue that a cell can be derived from, such as for example hematopoietic stem cells or neuronal stem cells. To the extent human cells are envisaged for use in the methods of the invention, it is preferred that such human cell is not obtained from a human embryo, in particular not via methods entailing destruction of a human embryo. On the other hand, human embryonic stem cells are at the skilled person's disposal such as taken from existent embryonic stem cell lines commercially available. Accordingly, the present invention may be worked with human embryonic stem cells without any need to use or destroy a human embryo. Alternatively, or instead of human embryonic stem cells, pluripotent cells that resemble embryonic stem cells such induced pluripotent stem (iPS) cells may be used, the generation of which is state of the art (Hargus G et al., 2010, Proc Natl Acad Sci USA, 107:15921-15926; Jaenisch R. and Young R., 2008, Cell 132:567-582; Saha K, and Jaenisch R., 2009, Cell Stem Cell 5:584-595).

In compounds according to the following formula: 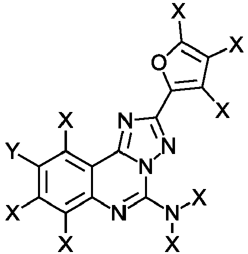 each X is H; and Y is halogen, preferably Y is selected from Cl, F, and Br.

In embodiments, Y is halogen, preferably Cl.

In an embodiment of the invention, the compound is

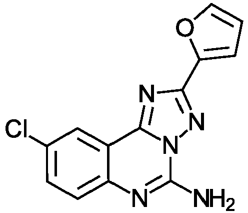

In various embodiments, said method is a method to differentiate a cell and wherein (a) the cell is a pluripotent stem cell; and/or (b) the cell differentiates into a cardiac progenitor cell, myoblast, hematopoietic stem cell or mesenchymal stem cell; and/or (c) the differentiation further comprises contacting the cell with at least one compound supporting differentiation.

Methods to differentiate cells involving the use of BMP activity are well-known in the art. For example, the use of BMP4 to generate cardiac progenitor cells or cardiac muscle cells from human stem cells is disclosed in Burridge et al. 2014, Zhang et al. 2015 and Rao et a 2016. In addition, BMPs and BMP mimetic compounds can be used specifically to influence the fate of stem and precursor cells. This "directed differentiation" of embryonic and/or inductive pluripotent stem cells into desired specialized target cells and tissues can involve BMPs or BMP mimetic compounds which are used for the induction of mesoderm. In the art there well-known protocols available to differentiate stem and progenitor cells to: a) cardiovascular precursor cells and myoblasts, which later form the cells of the blood vessels, the heart muscle and the skeletal muscle; b) hematopoietic stem cells, which form all cells of the blood; c) mesenchymal stem cells (MSCs), which are the starting point for cells of adipose tissue, bone and cartilage.

In embodiments, the method to differentiate a cell comprises converting a cell, preferably a stem cell, into a mesodermal cell, preferably a cardiac progenitor, cardiomyocyte, or hemangioblast. In alternative embodiments, the method to differentiate a cell comprises converting a cell, preferably a stem cell, into an ectodermal cell, preferably an epithelial stem cell. In other alternative embodiments, the method to differentiate a cell comprises converting a cell, preferably a stem cell, into an endodermal cell, preferably a hepatocyte progenitor or immature lung cell.

Stem cells are cells in multicellular organisms that can divide and differentiate into diverse specialized cell types and can self-renew to produce more stem cells that have the same property. A "pluripotent stem cell", as used herein, is a stem cell that has the potential to differentiate into any of the three germ layers: endoderm (e.g., interior stomach lining, gastrointestinal tract, the lungs), mesoderm (e.g., muscle, bone, blood, urogenital system), and ectoderm (e.g., epidermal tissues and nervous system). Pluripotent stem cells can give rise to any fetal or adult cell type. For the purposes of this disclosure a "pluripotent stem cell" may include a totipotent stem cell, which is a cell that can construct a complete, viable organism. These cells are produced from the fusion of an egg and sperm cell. Cells produced by the first few divisions of the fertilized egg are also totipotent. Pluripotent stem cells include but are not limited to embryonic stem (ES) cells, induced pluripotent stem cells (iPSC), and cells produced by somatic cell nuclear transfer (SCNT).

ES cells are totipotent stem cells derived from the inner cell mass of the blastocyst of an early-stage mammalian embryo. Methods of deriving mammalian ES cells (including whose that are not destroying an embryo) are well known in the art as are numerous established ES cell lines that may be used in conjunction with certain embodiments of this disclosure.

The term "(human) cardiac progenitor cell", as used herein, refers to a (human) progenitor cell that is committed to the cardiac lineage and that has the capacity to differentiate into all three cardiac lineage cells (cardiac muscle cells, endothelial cells and smooth muscle cells). The term "(human) cardiomyogenic progenitor cell", as used herein, refers to a human progenitor cell that is committed to the cardiac lineage and that predominantly differentiates into cardiac muscle cells (i.e., more than 10% of the differentiated cells, preferably more than 20%, 30%, 40%, 50%, 60%, 70%, 80% or 90% of the differentiated cells, derived from the progenitor cells are cardiac muscle cells). The term "cardiomyocyte" refers to a muscle cell of the heart (e.g. a cardiac muscle cell). A cardiomyocyte will generally express on its cell surface and/or in the cytoplasm one or more cardiac-specific marker. Suitable cardiomyocyte-specific markers include, but are not limited to, cardiac troponin I, cardiac troponin-C, tropomyosin, caveolin-3, GATA-4, myosin heavy chain, myosin light chain-2a, myosin light chain-2v, ryanodine receptor, and atrial natriuretic factor.

The term "myoblast", as used herein, refers to an embryonic cell in the mesoderm that differentiates to give rise to a muscle cell or myocyte. The term includes cells *in vivo* and cells cultured *ex vivo* regardless of whether such cells are primary or passaged.

"Hematopoietic stem cell", as used herein, refers to a cell, e.g., a bone marrow cell, or a fetal liver or spleen cell, which is capable of developing into all myeloid and lymphoid lineages and by virtue of being able to self-renew can provide long term hematopoietic reconstitution. Purified preparations of hematopoietic cells or preparations, such as bone marrow, which include other cell types, can be used in methods of the invention. Although not wishing to be bound by theory, it is believed that the hematopoietic stem cells home to a site in the recipient. Hematopoietic stem cells can be from fetal, neonatal, immature or mature animals. Stem cells derived from the cord blood of the recipient or the donor can be used in methods of the invention (see U.S. Patent 5,192,553, and U.S. Patent 5,004,681).

The phrase "mesenchymal stem cell", as used herein, refers to a stem cell of mesenchymal origin. Mesenchymal stem cells are multipotent stromal cells that can differentiate into a variety of cell types, including osteoblasts, chondrocytes and adipocytes.

In preferred embodiments, signaling pathway modulators comprise
1) Wnt/β-Catenin stimulators and GSK3 inhibitors, such as CHIR99021, BIO, kenpaullone, LiCl, Wnt ligands (e.g. Wnt-3a);
2) Wnt/β-Catenin inhibitors, such as
   - Tankyrase inhibitors (such as XAV939, NVP-TNKS656, Compound 25 and analogs, IWR-1, 53AH and analogs),
   - Porcupine inhibitors (such as IWP-2, IWP-4, Wnt-C59, LGK974 and analogs),
   - β-Catenin protein-protein-inhibitors (such as iCRT-14 and analogs, ICG-001, IQ-1, StAx-35R and analogs, TU-002, windorphen, carnosic acid),
   - diverse compounds (such as cardionogen-1, KY-02111, pyrvinium, SB-203580, TA-02, CCT251545 and anlogs, CCT031374), and
   - Dickkopf-1 (Dkk1)
3) modulators of non-canonical Wnt signaling (such as Wnt ligands/proteins),
4) TGFβ/Activin/Nodal stimulators (such as TGFβ isoforms, Activin A, Nodal),
5) TGFβ/Activin inhibitors (such as A83-01, SB431542, RepSox, LY-2157299, galunisertib, LY-2109761, GW788388, SB-525334, SB-505124, EW-7203 and analogs, (+)-R-ITD-1, ITD-ts and analogs),
6) Sonic hedgehog (Shh) signaling modulators, such as
   - stimulators: such as SAG, Smoothened, Hh-Ag1.5, purmorphamine, SHH (= ligand), and
   - inhibitors: such as Shh inhibitors (LDE225, cyclopamine, robotnikinin),
7) retinoic acid signaling modulators (such as retinoic acid, BMS-189453),
8) miscellaneous signaling pathway modulators, such as
   - PDGF, VEGF, FGF inhibitors (such as JNJ10198409, SU16F, SU5402, PD-173074),
   - VEGF signaling mimetics, such as ONO-1301, FGF signaling mimetics such as SUN-11602,
   - G-CSF mimetic: such as SB-247464,
   - EGFR inhibitors (such as erlotinib),
   - Notch signaling inhibitors (such as Compound E),
   - Hippo/Yap signaling modulators (such as cardiac glycoside digitoxin, C108, verteporfin, protoporphyrin IX, 9E, MST1 and MST2 inhibitors such as XMU-MP-1),
   - P38-MAPK inhibitors (such as SB-203580, SB-23906, skepinon-L etc.),
   - MAP/ERK/MEK inhibitors (such as PD0325901, SU1498, SC1 = Pluripotin, PD-184352),
   - PI3K inhibitors (such as LY294002),
   - ROCK inhibitors (such as Y27632, thiazovivin),
   - Raf inhibitors (such as ZM336372),
   - JAK inhibitors (such as JI1),
   - JNK inhibitor (such as SP600125),
   - IGF agonists (such as IGF-1) and IGF antagonists,
   - CaMKII inhibitors (such as KN62, KN93),
   - mTOR inhibitors (such as Rapamycin).

### Growth factors comprise

- Bone Morphogenetic Protein (BMP) isoforms (BMP-2, -3, -4, -5, -6, -7, -8, -9, -12, -13, -14),
- Thrombopoetin (TPO), erythropoetin (EPO), Colony-stimulating factors (such as G-CSF), interleukins (such as IL2, IL6, IL11), stem cell factor (SCF)/cKit-Ligand, HGF, VEGF, insulin, PDGF, thymosin β4, IGF1, neuregulin-1 (NRG1), FGFs (FGF-2, -3, -10, -11, -13, - 15), LIF, SHH, Wnt ligands, TGFβ-1, -2, -3.

Methods and protocols using BMPs and mimetics thereof are well-known in the art. In preferred embodiments, fibroblasts are transdifferentiated to cardiac muscle cells. Such methods and protocols are described in the art by Wang et al 2014, Cao et al 2016, Zhang et al., 2016.

The term "fibroblast" as used herein, refers to a type of cell that synthesizes the extracellular matrix and collagen, the structural framework (stroma) for animal tissues, and that plays a critical role in wound healing. Fibroblasts are the most common cells of connective tissue in animals.

The term "cardiac muscle cell", as used herein, refers to are the muscle cells (myocytes) that make up the cardiac muscle (heart muscle). Typical marker protein for cardiac muscle cells include but are not restricted to NPPB (natriuretic peptide B), MYL7 (myosin, light chain 7), NPPA (natriuretic peptide A), MYBPC3 (myosin binding protein C, cardiac), TNNT2 (troponin T type 2 (cardiac)), BMP10 (bone morphogenetic protein 10), TNNI3 (troponin I type 3 (cardiac)), MYH6 (myosin, heavy chain 6, cardiac muscle, alpha), ANKRD1 (ankyrin repeat domain 1 (cardiac muscle)), RD3L (retinal degeneration 3-like), SBK2 (SH3 domain binding kinase family, member 2), and MYL4 (myosin, light chain 4, alkali; atrial, embryonic).

In further alternative embodiments, said method is a method to reprogram a cell and wherein (a) the cell is a fibroblast; and/or (b) the cell reprograms into a pluripotent stem cell; and/or (c) the reprogramming further comprises contacting the cell with at least one compound selected from the group of Oct-4, Sox-2, Klf-4 or c-Myc.

"Reprogramming", as used herein, includes the transformation of an initially cell, for example a fibroblast, to different type of cell, such as a pluripotent stem cell. Protocols for the reprogramming of fibroblasts to pluripotent stem cells using BMPs or BMP mimetic compounds are disclosed in the art, for example by Chen et al., Cell Res. 2011 Jan; 21(1): 205-212. The reprogramming of fibroblasts to pluripotent stem cells using a BMP mimetic compound as described herein can be done using all Yamanaka factors (Oct-4, Sox-2, Klf-4 and c-Myc). However, in preferred embodiments, the BMP mimetic compound is only used in combination with Oct-4 and Sox-2 or in combination with Oct-4.

Additional references describing BMP mimetics in reprogramming of somatic cells to pluripotent cells include in the context of a) murine cells Chen et al. (Chen et al., Cell Res. 2011 Jan; 21(1): 205-212) and Samavarchi-Tehrani et al. (Samavarchi-Tehrani et al., Cell Stem Cell. 2010 Jul 2;7(1):64-77) and b) human cells Hayashi et al. (Hayashi, Y. et al., Proc Natl Acad Sci USA. 2016 Nov 15;113(46): 13057-13062).

In a second aspect, the present invention is directed to a compound of the invention or a pharmaceutically acceptable salt, solvate or prodrug thereof, as described above, for use in the treatment of osteoporosis, bone fracture, osteonecrosis, osteoarthritis, rheumatoid arthritis, spondylosis, anterior lumbar fusion or long bone fracture, wherein said prodrug is a phosphoric acid ester linked via a one-carbon-bridge.

The compound for use is thus defined as described above for the compounds used in the context of the methods for differentiating, transdifferentiating or reprogramming a cell.

According to certain embodiments, a compound, pharmaceutically acceptable salt, solvate or prodrug thereof for use in the treatment of osteoporosis, bone fracture, osteonecrosis, osteoarthritis, rheumatoid arthritis, spondylosis, anterior lumbar fusion or long bone fracture may be administered in the form of a pharmaceutical composition.

Thus, the present invention also provides a pharmaceutical composition comprising at least one compound, pharmaceutically acceptable salt, solvate or prodrug form thereof, as described herein, in an amount effective for treating a disorder, and a pharmaceutically acceptable vehicle or diluent, wherein said prodrug is a phosphoric acid ester linked via a one-carbon-bridge. The compositions of the disclosure may contain other therapeutic agents as described below, and may be formulated, for example, by employing conventional solid or liquid vehicles or diluents, as well as pharmaceutical additives of a type appropriate to the mode of desired administration (for example, excipients, binders, preservatives, stabilizers, flavors, etc.) according to techniques such as those well known in the art of pharmaceutical formulation.

The term "therapeutically effective amount" means the amount of the compound or pharmaceutical composition that will elicit the biological or medical response of a tissue, system, animal or human that is being sought.

The term "osteoporosis", as used herein, is defined as a systemic skeletal disease characterized by low bone mass and micro-architectural deterioration of bone tissue, with a consequent increase in bone fragility and susceptibility to fracture. The clinical definition of osteoporosis is a condition in which the bone mineral density (BMD) or bone mineral concentration (BMC) is greater than about 2.5 standard deviations (SD) below the mean of young healthy women. Severe osteoporosis is defined as having a BMD or BMC greater than about 2.5 SD below the mean of young healthy women and the presence of one or more fragility fractures. Since bone loss is not strictly confined to specific sites, osteoporosis can manifest itself in various ways including alveolar, femoral, radial, vertebral or wrist bone loss or fracture incidence, postmenopausal bone loss, severely reduced bone mass, fracture incidence or rate of bone loss.

The term "bone fracture", as used herein, means one of various physical injuries of a bone, based on a complete or incomplete disruption of the continuity of a bone, which are classified according to anatomical location (epiphyseal, metaphyseal, diaphyseal, intra-articular, proximal, midshaft, distal, etc.), degree of fracture (complete, incomplete), direction of fracture (transverse, oblique, spiral, longitudinal), presence of open wound (open, closed), number of fractures (simple, linear, segmental, comminuted, etc.), stability of fracture (stable, unstable), displacement of fracture, etc.

"Osteonecrosis", as used herein, is cellular death (necrosis) of bone components due to interruption of the blood supply. Without blood, the bone tissue dies and the bone collapses. If osteonecrosis/avascular necrosis involves the bones of a joint, it often leads to destruction of the joint articular surfaces.

The term "osteoarthritis", as used herein, is a type of joint disease that results from breakdown of joint cartilage and underlying bone. The most common symptoms are joint pain and stiffness. Other symptoms may include joint swelling, decreased range of motion, and when the back is affected weakness or numbness of the arms and legs. Unlike other types of arthritis, only the joints are typically affected. Causes include previous joint injury, abnormal joint or limb development, and inherited factors.

"Rheumatoid arthritis", as used herein, is a long-term autoimmune disorder that primarily affects joints. It typically results in warm, swollen, and painful joints. Most commonly, the wrist and hands are involved, with the same joints typically involved on both sides of the body. Rheumatoid arthritis may also result in a low red blood cell count, inflammation around the lungs, and inflammation around the heart.

The term "spondylosis", as used herein, refers to a broad term meaning degeneration of the spinal column from any cause. In the more narrow sense it refers to spinal osteoarthrosis, the age-related wear and tear of the spinal column, which is the most common cause of spondylosis. The degenerative process in osteoarthritis chiefly affects the vertebral bodies, the neural foramina and the facet joints (facet syndrome). If severe, it may cause pressure on nerve roots with subsequent sensory or motor disturbances, such as pain, paresthesia, and muscle weakness in the limbs.

"Anterior lumbar fusion" or "anterior lumbar interbody fusion (ALIF)", as used interchangeably herein, is similar to the posterior lumbar interbody fusion (PLIF), except that in the ALIF, the disc space is fused by approaching the spine through the abdomen instead of through the lower back. This surgery treatment is well-known in the art.

The term "BMP-responsive element", as used herein, refers to a nucleic sequence that can bind BMP, preferably BMP4, and subsequently increases the expression of a gene of interest. Such BMP-responsive elements can be found, for example, in the promoter sequence of Id-1 and are well-known in the art. Katagiri et al. (Katagiri, T. et al. Genes Cells. 2002 Sep;7(9):949-60) and Korchynskyi et al. (Korchynskyi O, ten Dijke P. J Biol Chem. 2002 Feb 15;277(7):4883-91. Epub 2001 Nov 29. 10.1074/jbc.M111023200) describe such element.

The term "contacting", as used herein in the context of mimetic compounds of interest and cells, refers generally to providing access of one component, reagent, analyte or sample to the cell. For example, contacting can involve mixing a solution comprising a BMP mimetic compound and a cell. The solution comprising the mimetic compound may also comprise another component or reagent, such as dimethyl sulfoxide (DMSO) or a detergent, which facilitates mixing, interaction, uptake, or other physical or chemical phenomenon advantageous to the contact between components, reagents, analytes and/or samples.

### EXAMPLES

### Methods

### Chemicals and agents

DAPI (Carl Roth)
DMH-1 (Selleck Chemicals)
DMSO (Agros Organics)
Formaldehyde (Carl Roth)
Gelatin solution 0,1 % (PAN Biotech)

### Media

| | | | |
|---|---|---|---|
| Cultivation medium: | 87% | DMEM | Thermo Fisher |
| | 10% | FBS | PAN Biotech |
| | 1% | GlutaMAX | Thermo Fisher |
| | 1% | NEAA | Thermo Fisher |
| | 1% | Pen Strep | Thermo Fisher |
| | 0,1 mM | 2-Mercaptoethanol | Thermo Fisher |
| | 15 ng/mL | LIF | |
| Differentiation medium (1): (D0, D4, D8) | 87% | DMEM | Thermo Fisher |
| | 10% | FBS | PAN Biotech |
| | 1% | GlutaMAX | Thermo Fisher |
| | 1% | NEAA | Thermo Fisher |
| | 1% | Pen Strep | Thermo Fisher |
| | 0,1 mM | 2-Mercaptoethanol | Thermo Fisher |
| Differentiation medium (2): (D3) | 93% | DMEM | Thermo Fisher |
| | 4% | FBS | PAN Biotech |
| | 1% | GlutaMAX | Thermo Fisher |
| | 1% | NEAA | Thermo Fisher |
| | 1% | Pen Strep | Thermo Fisher |
| | 0,1 mM | 2-Mercaptoethanol | Thermo Fisher |

### Buffer

| | | | |
|---|---|---|---|
| Phosphate buffer (1x): | 137 mM | NaCl | Carl Roth |
| | 2,7 mM | KCl | Carl Roth |
| | 10 mM | Na₂HPO₄ | Carl Roth |
| | 1,8 mM | KH₂PO₄ | Carl Roth |

### Devices

| | |
|---|---|
| Countess cell counter | Invitrogen |
| Echo^{®} Liquid Handler | Labcyte |
| EVOS XL Core Mikroskop | Life Technologies |
| ImageXpress Micro XL | Molecular Devices |
| Inkubator Hera Cell 150 | Thermo Fisher |
| Matrix pipette | Thermo Fisher |
| Microplate Washer ELx405 Select CW | BioTek |
| Multidrop Combi | Thermo Scientific |

### Cell line

| Cell line | Reporter | Organism | Publication |
|---|---|---|---|
| CGR8 | *α-Myh6-eGFP* | Mouse | Circulation 2003;107:1912-16 |

### Cell cultivation

Work with the pluripotent stem cells was performed under aseptic conditions in a sterile bench. All materials that came into contact with the cells were previously autoclaved or disinfected. The cells were cultured in an incubator at 37 ° C and 5% COz.

### Thawing of pluripotent stem cells

The cells which were cryopreserved at -150 ° C were carefully transferred to cultivation medium after rapid thawing in a water bath and then centrifuged (25 ° C, 3 min, 1200 rpm). The supernatant was then removed and the cells were plated out at a concentration of 20,000 cells per mL.

### Cultivation of pluripotent stem cells

The cells were passed every two to three days to a confluence of about 60-80%. For this purpose, the surface on which the cells were to be further cultivated was first coated with 0.1% gelatin solution and incubated at RT. After the cultivation medium was removed from the cells, the cells were first washed with buffer solution before they were incubated with trypsin-EDTA solution for 5 min at 37 ° C. The cells were then singulated and incubated at 37 ° C for 2 min. The reaction was then stopped by addition of cultivation medium. The cell trypsin suspension was transferred to a centrifuge tube and centrifuged (25 ° C, 3 min, 1200 rpm). The supernatant was removed and the cell pellet was resuspended in cultivation medium. The cell count was determined and a cell suspension of the desired cell concentration was prepared in a centrifuge tube. After the gelatin solution was removed, the cells were plated on the new culture surface and further cultured in the incubator (37 ° C, 5% COz).

### Freezing of pluripotent stem cells

In order to cryopreserve the cells, the cell pellet was resuspended in the freezing medium during the respective cell passage. For each freezing tube, the cell concentration was adjusted to 1×10⁶ cells/mL and the freezing tubes were first cooled step by step (1 ° C/min) in a freezing container to -80 ° C before they were stored the next day at -150 ° C.

### Cell count

The cell count was determined either with an automatic cell counter or with the aid of the Neubauer counting chamber. For this purpose, 10 µl of cell suspension was mixed with 10 µl of trypan blue solution and 10 µl of this suspension was added to a counting chamber and counted.

### BMP mimetics screening assay

To investigate new activators of the BMP signaling pathway, a stem cell-based high-content assay was developed using murine embryonic stem cells of the CGR8 cell line which had been stably transfected with the promoter-reporter construct α-MHC-GFP. At day 0 of the assay, 500 pluripotent stem cells were seeded in a 25 µL serum-containing medium (10% FBS) on a previously gelatin-coated 384-well cultivation plate and cultured for eleven days (37 ° C, 5% COz). On day 3, libraries of low molecular weight substances were transferred to the cells with the aid of the Echo^{®} Liquid Handler, and serum-containing medium (4% FBS) was then added to each well which additionally contained the selective BMP inhibitor DHM1 at a concentration of 0.5 µM. After 24 h, on day 4, the medium was aspirated and replaced by 75 µL of serum-containing medium (10% FBS) per well. This step was repeated on
day 8. On day 11, the cells were first subjected to formaldehyde fixation (16% formaldehyde solution) and DAPI stained (1: 250), and after three washings with PBS fluorescence microscopy was measured in the channels FITC (= GFP) and DAPI. The analysis was then carried out using MetaXpress 5.3 using multiwavelength cell scoring.

### Example 1: Establishing a high content screening assay to screen for BMP mimetic compounds

Figures 1-3 summarize the finding of the present inventors that a BMP-dependent time window during mESC differentiation can be identified. Especially the differentiation profile of the pro-cardiogenic effect of the unselective BMP inhibitor dorsomorphine (DM) versus the selective analogue DMH1 during the mESC differentiation provided important information to identify a highly BMP-dependent time window (Fig. 1). Between days 3-5 of the differentiation, both DM and DMH1 are cardiogenic, whereas between day 4-6, only DM is cardiogenic, which may be explained by its pronounced inhibition of TGFβ. It is also shown that the selective cardiomyogenesis induced by DMH1 can be antagonized by BMP4 in a dose-dependent manner (Fig. 2). The low molecular weight compound isoliquiritigenin (Vrijens et al. 2013), which is a BMP4 mimetic compound in the absence of BMPs, also shows a dose-dependent effect. However, the effect of isoliquiritigenin is weak and said agent is a chalcone and thus pleiotropic in its biological activity.

Figure 3 summarizes the observed profile of the three most important signal pathways, which can be observed in a time-dependent manner and independent of each other during the differentiation of mESCs.

### Example 2: High content screening of BMP mimetic compounds

Based on the above-described data, a screening for low-molecular-weight BMP mimetics was carried out at a time frame of days 3-4 after the start of stem cell cultivation/differentiation. In parallel, cardiomyogenesis was stimulated with DMH1 (0.5 µM) in the same time frame (days 3-4) and the cardiomyogenesis inhibitory effect was quantified using high-content imaging in the 384-well format. Since the present screening assay uses a "negative readout", the probability of identifying false-positive hits that only affect the viability of the cells (or are toxic) is significant. Thus, the primary readout on cardiomyogenesis was combined with a cell count (DAPI staining). Subsequently, false positive hits and artefacts were eliminated by means of a visual control.

This workflow was carried out with the commercially available LOPAC substance library (obtained from Sigma-Aldrich) of pharmacologically active drugs. A total of 1408 substances were screened in the new test system (Figure 4). Substances with an activity > 50% inhibition (= 57 hits) were tested for wrong-positive activity by toxicity and artefacts (= 33 hits). For these 33 hits, a dose-dependent BMP mimetic effect should be detected so that IC₅₀ values were determined over a dose range of 5-0.01 µM. The remaining 33 hits were also checked visually for artefacts. 10 hits demonstrate such a dose-dependent effect and have thus been validated as new BMP mimetic compounds.

In further validation experiments, for three of these 10 hits, significant selectivity for the BMP signaling pathway could be demonstrated against other closely related TGFβ family signaling pathways on ligands as well as on the canonical Wnt signaling pathway. Particularly, the substance CGS-15943 demonstrated an attractive profile (Fig. 5). It should be pointed out that all 10 validated hits from the LOPAC screen (Fig. 4) are effective as new BMP mimetics in the stem cell context of mesodermal differentiation.

## Claims

1. An *in vitro* method for differentiating, transdifferentiating or reprogramming a cell, said method comprising:
contacting the cell with a compound 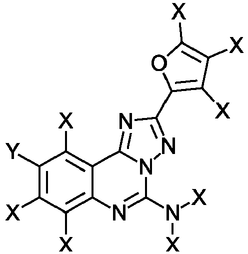 wherein
XisH;
Y is halogen, wherein the cell differentiates into_a mesenchymal cell that can differentiate into osteoblasts and chondrocytes.

2. The *in vitro* method according to claim 1, wherein the compound is

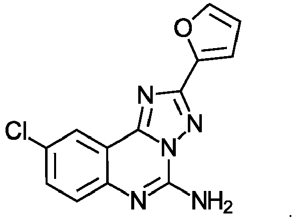

3. The *in vitro* method according to one of claims 1 to 2, wherein
(I) the method differentiates the cell and wherein
(a) the cell is a pluripotent stem cell; and/or
(b) the cell differentiates into a multipotent cell, preferably a cardiac progenitor cell, myoblast, hematopoietic stem cell or mesenchymal stem cell; and/or
(c) the differentiation further comprises contacting the cell with at least one compound supporting differentiation;
(II) said method is a method to transdifferentiate a cell and wherein
(a) the cell is a fibroblast; and/or
(b) the cell transdifferentiates into a cardiac muscle cell; and/or
(c) the transdifferentiation further comprises contacting the cell with at least one compound supporting transdifferentiation; or
(III) said method is a method to reprogram a cell and wherein
(a) the cell is a fibroblast; and/or
(b) the cell reprograms into a pluripotent stem cell; and/or
(c) the reprogramming further comprises contacting the cell with at least one compound selected from the group of Oct-4, Sox-2, Klf-4 or c-Myc.

4. Compound for use in the treatment of osteoporosis, bone fracture, osteonecrosis, osteoarthritis, rheumatoid arthritis, spondylosis, anterior lumbar fusion or long bone fracture by differentiating, transdifferentiating or reprogramming cells, wherein the cells differentiate into mesenchymal stem cells that can differentiate into osteoblasts and chondrocytes, **characterized in that** the compound is 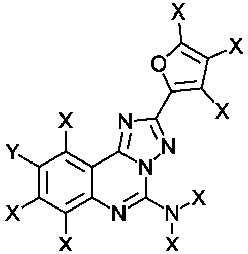 wherein
X is H;
Y is halogen.

5. Compound for use according to claim 4, wherein the compound is

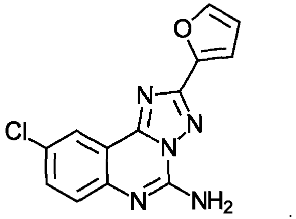

6. Compound for use according to claim 4 or 5 in the form of a pharmaceutically acceptable salt, solvate or prodrug thereof, wherein said prodrug is a phosphoric acid ester linked via a one-carbon-bridge.

7. Compound for use according to claim 6, wherein the one-carbon-bridge is a methylene group or an alkylmethyl group linker.

## Patentansprüche

1. In vitro Verfahren zur Differenzierung, Transdifferenzierung oder Reprogrammierung einer Zelle, wobei das Verfahren umfasst:
Kontaktieren der Zelle mit einer Verbindung 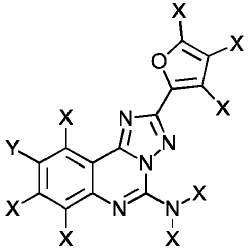
bei der
X H ist;
Y Halogen ist, wobei sich die Zelle in eine mesenchymale Zelle differenziert, die zu Osteoblasten und Chondrozyten differenzieren kann.

2. Das in vitro Verfahren nach Anspruch 1, bei dem die Verbindung 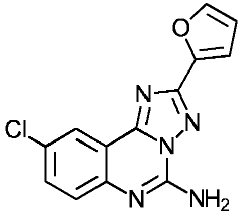 ist.

3. Das in vitro Verfahren nach einem der Ansprüche 1 bis 2, bei dem
I) das Verfahren die Zelle differenziert und bei dem
(a) die Zelle eine pluripotente Stammzelle ist; und/oder
(b) die Zelle zu einer multipotenten Zelle differenziert, bevorzugt einer kardialen Vorläuferzelle, Myoblast, hematopoietischen Stammzelle oder mesenchymalen Stammzelle und/oder
(c) die Differenzierung weiter das Kontaktieren der Zelle mit wenigstens einer Verbindung umfasst, die die Differenzierung unterstützt;
(II) das Verfahren ein Verfahren zum Transdifferenzierung einer Zelle ist und bei dem
(a) die Zelle ein Fibroblast ist und/oder
(b) die Zelle zu einer kardialen Muskelzelle transdifferenziert und/oder
(c) die Transdifferenzierung weiter das Kontaktieren der Zelle mit wenigstens einer Verbindung umfasst, die die Transdifferenzierung unterstützt oder
(III) das Verfahren ein Verfahren zum Reprogrammieren einer Zelle ist und bei dem
(a) die Zelle ein Fibroblast ist und/oder
(b) die Zelle zu einer pluripotenten Stammzelle reprogrammiert wird und/oder
(c) das Reprogrammieren weiter das Kontaktieren der Zelle mit wenigstens einer Verbindung umfasst, die aus der Gruppe von Oct-4, Sox-2, Klf-4 oder c-Myc ausgewählt ist.

4. Verbindung zur Verwendung bei der Behandlung von Osteoporose, Knochenbrüchen, Osteonekrosen, Osteoarthritis, rheumatoider Arthritis, Spondylose, vorderer Lumbarfusion oder Fraktur langer Knochen durch Differenzierung, Transdifferenzierung oder Reprogrammierung von Zellen, wobei die Zellen zu mesenchymalen Stammzellen differenzieren, die in Osteoblasten und Chondrozyten differenzieren können, **dadurch gekennzeichnet, dass** die Verbindung 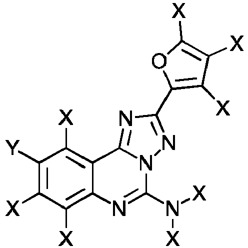 ist, bei der
X H ist;
Y Halogen ist.

5. Verbindung zur Verwendung nach Anspruch 4, wobei die Verbindung 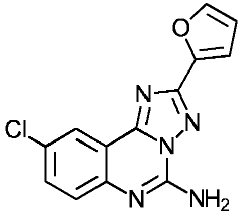 ist.

6. Verbindung zur Verwendung nach Anspruch 4 oder 5 in der Form eines
pharmazeutisch annehmbaren Salzes, Solvats oder Vorläuferverbindung davon, wobei die Vorläuferverbindung ein Phosphorsäureester ist, der über eine Ein-Kohlenstoff-Brücke verbunden ist.

7. Verbindung zur Verwendung nach Anspruch 6, wobei die Ein-Kohlenstoff-Brücke eine Methylengruppe oder ein Alkylmethyl-Gruppen-Verbinder ist.

## Revendications

1. Procédé, in vitro, de différenciation, de transdifférenciation ou de reprogrammation d'une cellule, ledit procédé comprenant:
mise en contact de la cellule avec un composé 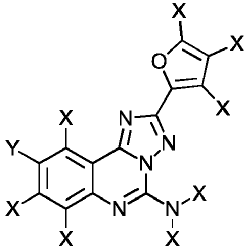 où
X est H;
Y est un halogène, où la cellule se différencie en une cellule mésenchymateuse qui peut se différencier en ostéoblastes et chondrocytes.

2. Procédé, in vitro, selon la revendication 1, dans lequel le composé est

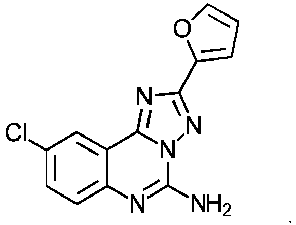

3. Procédé, in vitro, selon l'une des revendications 1 à 2, dans lequel
(I) le procédé permet de différencier la cellule et dans lequel
(a) la cellule est une cellule souche pluripotente; et/ou
(b) la cellule se différencie en une cellule multipotente, de préférence une cellule progénitrice cardiaque, un myoblaste, une cellule souche hématopoïétique ou une cellule souche mésenchymateuse; et/ou
(c) la différenciation comprend en outre la mise en contact de la cellule avec au moins un composé favorisant la différenciation;
(II) ladite procédé est une procédé de transdifférenciation d'une cellule et dans laquelle
(a) la cellule est un fibroblaste; et/ou
(b) la cellule se transdifférencie en une cellule de muscle cardiaque; et/ou
(c) la transdifférenciation comprend en outre la mise en contact de la cellule avec au moins un composé favorisant la transdifférenciation; ou
(III) ladite procédé est une procédé de reprogrammation d'une cellule et dans laquelle
(a) la cellule est un fibroblaste; et/ou
(b) la cellule se reprogramme en une cellule souche pluripotente; et/ou
(c) la reprogrammation comprend en outre la mise en contact de la cellule avec au moins un composé choisi dans le groupe des Oct-4, Sox-2, Klf-4 ou c-Myc.

4. Composé destiné à être utilisé dans le traitement de l'ostéoporose, des fractures osseuses, de l'ostéonécrose, de l'ostéoarthrite, de la polyarthrite rhumatoïde, de la spondylose, de la fusion lombaire antérieure ou de la fracture des os longs pour la différenciation, la transdifférenciation ou la reprogrammation des cellules, dans lequel les cellules se différencient en cellules souches mésenchymateuses qui peuvent se différencier en ostéoblastes et en chondrocytes,
**caractérisé en ce que** le composé est 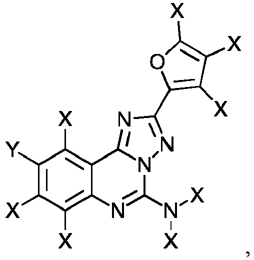 où
X est H;
Y est un halogène.

5. Composé à utiliser selon la revendication 4, dans lequel le composé est

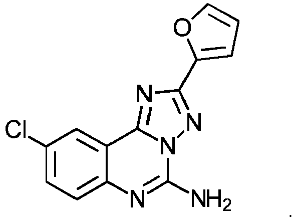

6. Composé à utiliser selon la revendication 4 ou 5 sous la forme d'un sel, d'un solvate ou d'un promédicament pharmaceutiquement acceptable de celui-ci, qui est un ester d'acide phosphorique lié par un pont à un carbone.

7. Composé à utiliser selon la revendication 6, dans lequel le pont à un carbone est un groupe méthylène ou un lieur de groupe alkylméthyle.
